# EUROPEAN PATENT APPLICATION

(11) **EP 4 782 810 A1**
(43) Date of publication of application: **29.07.2026**
(21) Application number: 24896204.5
(22) Date of filing: 05.11.2024
(51) Int. Cl.: G01M 99/00, G16H 10/40

(54) **INSTRUMENT STATE DETERMINATION METHOD AND APPARATUS, COMPUTER DEVICE, AND STORAGE MEDIUM**

(30) Priority: 29.11.2023 CN 202311626657
(71) Applicant: Shenzhen Yhlo Biotech Co., Ltd., Shenzhen, Guangdong 518116 (CN)
(72) Inventor: HUANG, Jingbo, Shenzhen, Guangdong 518116 (CN); LI, Guihui, Shenzhen, Guangdong 518116 (CN); ZHANG, Fuxing, Shenzhen, Guangdong 518116 (CN)
(74) Representative: Noble, Nicholas
(86) International application number: PCT/CN2024/129956
(87) International publication number: WO 2025/113113

(57) **Abstract**

The present application discloses an instrument state determination method and apparatus, a computer device, and a storage medium. The method can be applied to the technical field of computers, and specifically may comprise: when a self-quality-control reagent has been provided in a target instrument, using the self-quality-control reagent to perform self-quality-control detection on the target instrument to obtain a target detection result of the self-quality-control detection and determining the status of the target instrument on the basis of the target detection result of the self-quality control detection. The scheme can improve the accuracy and reliability of the determined instrument status.

## Description

### TECHNICAL FIELD

This application relates to the field of computer technology, and specifically to a method and an apparatus for determining instrument status, a computer device, and a storage medium.

### BACKGROUND

The field of in vitro diagnostic analyzers is used to detect and evaluate biological samples to help users diagnose diseases, monitor treatment progress, and provide health management advice.

Traditional in vitro diagnostic analyzers rely on supporting test project reagents to perform cumbersome manual quality control (QC) procedures to determine instrument status, which is time-consuming, labor-intensive and costly. In addition, manual operations are prone to errors, thus resulting in low accuracy and reliability of the determined instrument status.

### SUMMARY

Based on this, it is necessary to address the above technical problem by providing a method and an apparatus for determining instrument status, a computer device, and a storage medium that can improve the accuracy and reliability of the determined instrument status.

In a first aspect, the present application provides a method for determining instrument status, including:
performing a self-quality control (QC) test on a target instrument using a self-quality control (QC) reagent to obtain a target test result of the self-QC test, in a case where the self-QC reagent is installed in the target instrument; and
determining a status of the target instrument based on the target test result of the self-QC test.

In one of the embodiments, determining the status of the target instrument based on the target test result of the self-QC test includes:
determining the status of the target instrument to be normal, in a case where the target test result of the self-QC test follows a Westgard rule; and
determining the status of the target instrument to be abnormal, in a case where the target test result of the self-QC test does not follow the Westgard rule.

In one of the embodiments, the method further includes:
obtaining a mean of multiple historical test results of the self-QC test, in the case where the target test result of the self-QC test does not follow the Westgard rule;
comparing the target test result of the self-QC test with the mean to obtain multiple comparison results; and
outputting a target abnormality indicator based on the multiple comparison results.

In one of the embodiments, outputting the target abnormality indicator based on the multiple comparison results includes:
obtaining multiple deviation ranges, the multiple deviation ranges being determined based on a standard deviation of the mean; and
outputting the target abnormality indicator based on a preset correspondence and the number of comparison results indicating that the deviation range is exceeded, wherein the preset correspondence refers to a correspondence between the number of the comparison results indicating that the deviation range is exceeded, and a candidate abnormality indicator.

In one of the embodiments, the candidate abnormality indicator includes a warning indicator and an out-of-control indicator; and outputting the target abnormality indicator based on the preset correspondence and the number of the comparison results indicating that the deviation range is exceeded includes:
outputting the warning indicator when the number of the comparison results indicating that the deviation range (*µ* - 2*σ*, *µ* + 2*σ*) is exceeded is less than a preset quantity threshold, wherein *µ* denotes the mean, and *σ* denotes the standard deviation; and
outputting the out-of-control indicator when the number of the comparison results indicating that the deviation range (*µ* - 2*σ*, *µ* + 2*σ*) is exceeded is greater than or equal to the preset quantity threshold.

In one of the embodiments, performing the self-QC test on the target instrument using the self-QC reagent to obtain the target test result of the self-QC test includes:
performing the self- QC test on the target instrument by adopting a preset method and using the self-QC reagent to obtain the target test result of the self-QC test, wherein the preset method is one of a competitive method, a sandwich method, or an indirect method.

In a second aspect, the present application provides an apparatus for determining instrument status, including:
a detection module configured to perform a self-quality control (QC) test on a target instrument using a self-quality control (QC) reagent to obtain a target test result of the self-QC test, in a case where the self-QC reagent is installed in the target instrument; and
a determination module configured to determine a status of the target instrument based on the target test result of the self-QC test.

In a third aspect, the present application provides a computer device, including a memory and a processor. The memory has a computer program stored thereon, and the processor, when executing the computer program, implements steps of the method for determining instrument status above.

In a fourth aspect, the present application provides a computer-readable storage medium, having a computer program stored thereon. The computer program, when executed by a processor, causes the processor to implement steps of the method for determining instrument status above.

In a fifth aspect, the present application provides a computer program product, including a computer program. The computer program, when executed by a processor, implements steps of the method for determining instrument status above.

In the method and apparatus for determining instrument status, the computer device, and the computer-readable storage medium above, in the case where the self-QC reagent is installed in the target instrument, the self-QC test is performed on the target instrument using the self-QC reagent to obtain the target test result of the self-QC test, and then the status of the target instrument is determined based on the target test result of the self-QC test. Compared with the in vitro diagnostic analyzers in the prior art, which rely on the cumbersome manual QC procedures to determine instrument status thus resulting the lower accuracy and reliability of the determined instrument status, the method for determining instrument status of the present application, in which the self-QC reagent installed in the instrument is used to automatically perform the self-QC test on the target instrument and the entire process of the detection is automatically executed by a computer, greatly improves the accuracy and reliability of the ultimately determined instrument status.

### BRIEF DESCRIPTION OF THE DRAWINGS

FIG. 1 is a schematic flowchart of a method for determining instrument status according to an embodiment;
FIG. 2 is a schematic flowchart of outputting a target abnormality indicator according to an embodiment;
FIG. 3 is a schematic flowchart of outputting the target abnormality indicator according to another embodiment;
FIG. 4 is a schematic flowchart of the method for determining instrument status according to another embodiment;
FIG. 5 is a block diagram showing a structure of an apparatus for determining instrument status according to an embodiment;
FIG. 6 is a view showing an internal structure of a computer device according to an embodiment.

### DETAILED DESCRIPTION OF THE EMBODIMENTS

To make the objectives, technical solutions, and advantages of this application clearer and better understood, the application will be further described in detail below with reference to the accompanying drawings and embodiments. It should be understood that the specific embodiments described herein are only used to explain this application but are not intended to limit the application.

Traditional in vitro diagnostic analyzers rely on supporting test project reagents to perform cumbersome manual quality control (QC) procedures to determine instrument status, which is time-consuming, labor-intensive and costly. In addition, manual operations are prone to errors, thus resulting in low accuracy and reliability of a determined instrument status. In view of these, the embodiments of this application provide a method for determining instrument status to solve the aforementioned technical problems.

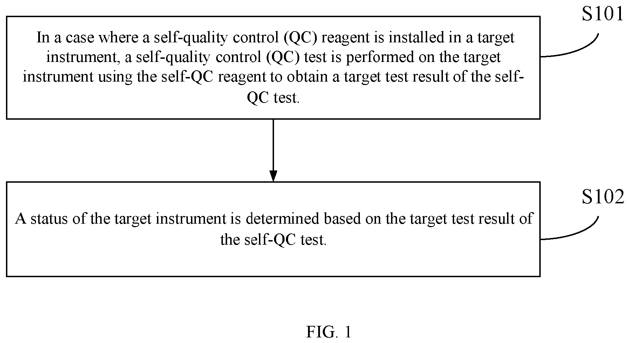

In an embodiment, FIG. 1 is a schematic flowchart of a method for determining instrument status according to an embodiment of this application. Taking this method applied to a server as an example for description, the method includes the following steps.

In S101, in a case where a self-quality control (QC) reagent is installed in a target instrument, a self-quality control (QC) test is performed on the target instrument using the self-QC reagent to obtain a target test result of the self-QC test.

Optionally, the target instrument can be an instrument designated for the self-QC test. The self-QC reagent may be a reagent installed on the target instrument to control target instrument to automatically perform the self-QC test. It should be noted that existing instruments cannot be installed with self-QC reagents, therefore they cannot automatically perform self-QC tests. Existing instruments can only be installed with QC reagents that cannot perform the self-QC automatically.

Optionally, a self-QC is a function that should be achieved by an appropriate control, and is mainly used to detect and control the precision of conventional instruments in a laboratory, and to detect changes in their accuracy, thereby improving the testing accuracy and reliability of the conventional instruments in the laboratory.

Specifically, after the self-QC reagent is installed in the target instrument, the target instrument is controlled to automatically perform the self-QC test using the self-QC reagent to obtain the target test result of the self-QC test.

In S102, a status of the target instrument is determined based on the target test result of the self-QC test.

Optionally, there is a preset correspondence between the target test result of the self-QC test and the status of the target instrument. After the target test result of the self-QC test is determined, the status of the target instrument can be obtained quickly and accurately based on the preset correspondence, which enables the rapid detection of instrument abnormalities and the timely execution of corresponding troubleshooting operations, thereby improving laboratory efficiency.

In the above method for determining instrument status, in the case where the self-QC reagent is installed in the target instrument, the self-QC test is performed on the target instrument using the self-QC reagent to obtain the target test result of the self-QC test, and then the status of the target instrument is determined based on the target test result of the self-QC test. Compared with the in vitro diagnostic analyzers in the prior art, which rely on the cumbersome manual QC procedures to determine instrument status thus resulting the lower accuracy and reliability of the determined instrument status, the method for determining instrument status of the present application, in which the self-QC reagent installed in the instrument is used to automatically perform the self-QC test on the target instrument and the entire process of the detection is automatically executed by a computer, greatly improves the accuracy and reliability of the ultimately determined instrument status.

Based on the above embodiments, it can be determined whether the status of the target instrument is normal by judging whether the target test result of the self-QC test follows a Westgard rule.

Optionally, the Westgard rule is a quality control (QC) rule that helps laboratories detect and assess errors and variability in the analytical process. The Westgard rule determines whether test results are within an acceptable range by setting a series of control limits for laboratory tests.

In the case where the target test result of the self-QC test follows the Westgard rule, the status of the target instrument is determined to be normal. Optionally, in the case where the target test result of the self-QC test follows the Westgard rule, it indicates that the error of the target test result is relatively small or almost negligible, and in this case, the status of the target instrument is determined to be normal.

In the case where the target test result of the self-QC test does not follow the Westgard rule, the status of the target instrument is determined to be abnormal. Optionally, in the case where the target test result of the self-QC test does not follow the Westgard rule, it indicates that the error of the target test result is relatively large, and in this case, the status of the target instrument is determined to be abnormal.

Optionally, in the case where the target test result of the self-QC test does not follow the Westgard rule, multiple historical test results of the self-QC test can be obtained, and a corresponding target abnormality indicator can be outputted based on the multiple historical test results. In a method: obtain the mean of the multiple historical test results of the self-QC test; compare the target test results of the self-QC test with the mean to obtain multiple comparison results; output the target abnormality indicator based on the multiple comparison results. In another method: obtain a root mean square (RMS) value of the multiple historical test results of the self-QC test; compare the target test results of the self-QC test with the RMS value to obtain multiple comparison results; output the target abnormality indicator based on these multiple comparison results.

It can be understood that determining whether the target instrument status is normal base on the Westgard rule in this embodiment is straightforward and easy to operate, laying the foundation for determining the type of the abnormal status of the target instrument.

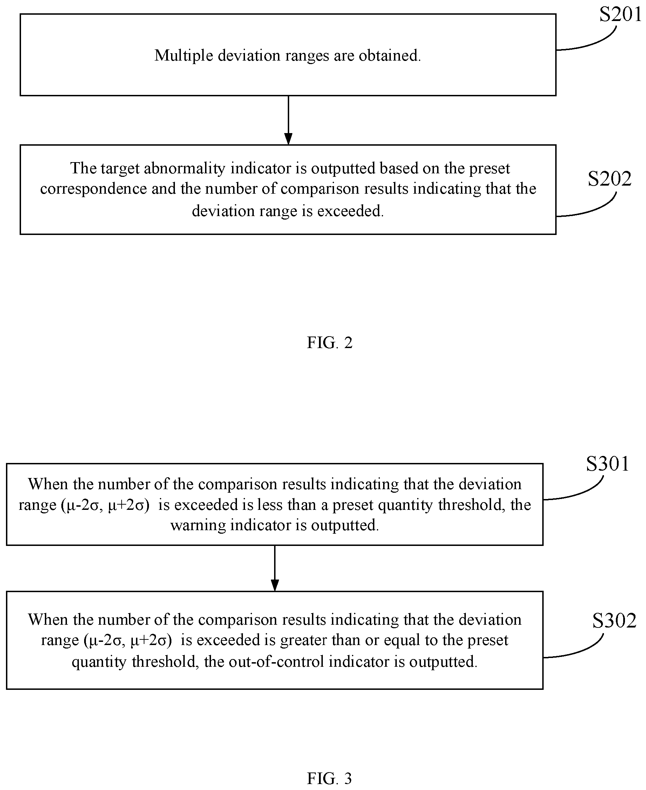

Based on the above embodiments, the step of outputting the target abnormality indicator is broken down and detailed in FIG. 2. Optionally, as shown in FIG. 2, the implementation process includes following steps.

In S201, multiple deviation ranges are obtained.

Optionally, the multiple deviation ranges are determined based on a standard deviation of the mean. Usually, the deviation range is the range represented by positive and negative multiples of the standard deviation of the mean. For example, if the standard deviation is *σ* and the multiple is 2, the deviation range is -2*σ* to +2*σ*.

In S202, the target abnormality indicator is outputted based on the preset correspondence and the number of comparison results indicating that the deviation range is exceeded.

Optionally, the preset correspondence refers to the correspondence between the number of the comparison results indicating that the deviation range is exceeded and a candidate abnormality indicator.

Optionally, count the number of the comparison results indicating that the deviation range is exceeded, and determine the type of abnormal status of the target instrument based on the preset correspondence, and output the corresponding target abnormality indicator. For example, if the preset correspondence is: when one test result falls outside the range of mean ± 2*σ*, a warning indicator is outputted; when two consecutive test results fall outside the range of mean ± 2*σ*, an out-of-control indicator is outputted. Based on the target test result of the self-QC test, combined with the counted number of the comparison results indicating that the deviation range is exceeded and the preset correspondence, the corresponding target abnormality indicator can be outputted.

It can be understood that in this embodiment, the whole process, which determines the type of target abnormality indicator to be outputted based on the number of the comparison results indicating that the deviation range is exceeded and the preset correspondence, is automatically executed by a computer, thereby greatly improving the accuracy and reliability of determining the type of target abnormality indicator.

Based on the above embodiments, the step of outputting the target abnormality indicator are further broken down and detailed in FIG 3. Optionally, the candidate abnormality indicator includes a warning indicator and an out-of-control indicator. As shown in FIG. 3, the implementation process includes the following steps.

In S301, when the number of the comparison results indicating that the deviation range (*µ* - 2*σ*, *µ* + 2*σ*) is exceeded is less than a preset quantity threshold, the warning indicator is outputted.

Where *µ* denotes the mean, and *σ* denotes the standard deviation.

Optionally, the deviation range (*µ* - 2*σ*, *µ* + 2*σ*) being exceeded indicates that an error of the target test result is relatively large. In this case, the status of the target instrument is determined to be abnormal. When the number of the comparison results indicating that the deviation range (*µ* - 2*σ*, *µ* + 2*σ*) is exceeded is less than a preset quantity threshold, it indicates that the number of the target test results with large errors is relatively small, and in this case, the warning indicator is outputted.

In S302, when the number of the comparison results indicating that the deviation range (*µ* - 2*σ*, *µ* + 2*σ*) is exceeded is greater than or equal to the preset quantity threshold, the out-of-control indicator is outputted.

Optionally, when the number of the comparison results indicating that the deviation range (*µ* - 2*σ*, *µ* + 2*σ*) is exceeded is greater than or equal to the preset quantity threshold, it indicates that the number of the target test results with large errors is relatively large, and in this case, the out-of-control indicator is outputted.

It should be noted that regarding the deviation range, a larger range, such as (*µ* - 3*σ*, *µ* + 3*σ*) or (*µ* - 4*σ*, *µ* + 4*σ*), indicates a relatively large allowable error. A smaller range, such as (*µ* - *σ*, *µ* + *σ*), indicates a relatively small allowable error. The deviation range and the preset quantity threshold can be adjusted according to precision requirements to obtain different classifications of abnormality indicators.

It can be understood that this embodiment provides a method for determining the type of abnormality indicator, which is straightforward simple, easy to implement, and highly flexible.

Based on the above embodiments, a detection method needs to be selected for the process of performing the self-QC test on the target instrument using the self-quality control (QC) reagent to obtain the target test result of the self-QC test. That is, the self-QC test is performed on the target instrument adopting a preset method and using the self-quality control (QC) reagent to obtain the target test result of the self-QC test. The preset method is one of a competitive method, a sandwich method, or an indirect method. Preferably, the preset method is the competitive method.

The competitive method is used to detect a concentration of a specific antigen or antibody in a sample. This method is based on a competitive binding between the antigen and a labeled substance (e.g., an antibody labeled with a radioactive isotope or enzyme). The antigen or antibody in the sample competes with the labeled substance for binding, thereby affecting the degree of binding of the labeled substance. By measuring the reduction in the binding of the labeled substance, the concentration of the specific antigen or antibody in the sample can be determined.

The indirect method is a common immunoassay method used to detect and measure the presence and concentration of a specific antibody in a sample. This method is based on the specificity of the binding between the specific antibody and an antigen to be detected. First, the antigen to be tested binds with a known antibody to form a complex. Then, a secondary antibody (typically an anti-animal antibody labeled with a fluorescent agent or enzyme) that binds to the known antibody is introduced. By detecting the signal of the secondary antibody, the presence and concentration of the specific antibody in the sample can be determined.

The sandwich method is used to detect and measure the presence and concentration of a specific antigen in a sample. This method is based on the simultaneous binding of two specific antibodies to the specific antigen, forming a sandwich structure. First, a specific antibody is coated on a test plate to bind with the antigen to be detected. Then, a second specific antibody is introduced, which binds to another epitope of the antigen to be detected and reacts with a substrate to produce a detectable signal. By measuring the intensity of the signal, the presence and concentration of the specific antigen in the sample can be determined.

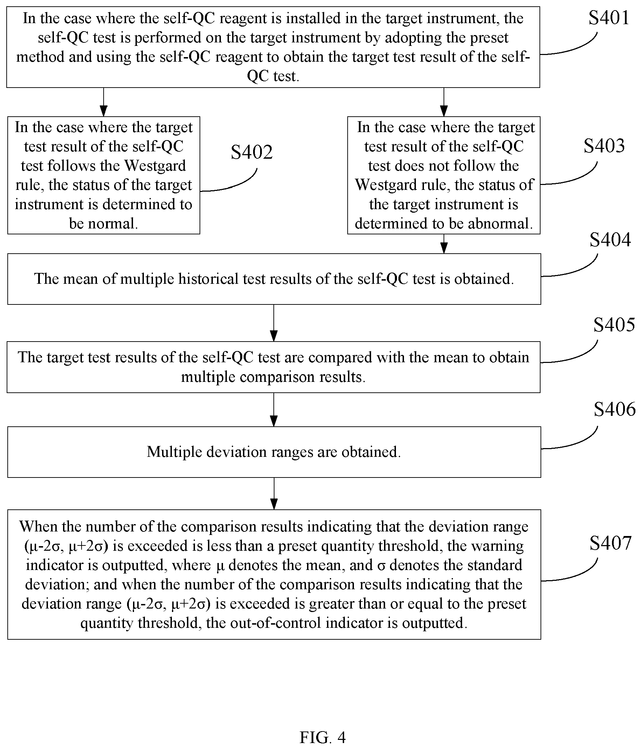

In an embodiment, FIG. 4 shows a schematic flowchart of the method for determining instrument status according to another embodiment. As shown in FIG. 4, the specific implementation process includes following stages.

In S401, in the case where the self-QC reagent is installed in the target instrument, the self-QC test is performed on the target instrument by adopting the preset method and using the self-QC reagent to obtain the target test result of the self-QC test.

Optionally, the preset method is one of a competitive method, a sandwich method, or an indirect method. Preferably, the preset method is the competitive method.

In S402, in the case where the target test result of the self-QC test follows the Westgard rule, the status of the target instrument is determined to be normal; and in the case where the target test result of the self-QC test does not follow the Westgard rule, the status of the target instrument is determined to be abnormal.

In S403, in the case where the target test result does not follow the Westgard rule, the mean of multiple historical test results of the self-QC test is obtained.

In S404, the target test results of the self-QC test are compared with the mean to obtain multiple comparison results.

In S405, multiple deviation ranges are obtained.

Optionally, the multiple deviation ranges are determined based on the standard deviation of the mean.

In S406, when the number of the comparison results indicating that the deviation range (*µ* - 2*σ*, *µ* + 2*σ*) is exceeded is less than a preset quantity threshold, the warning indicator is outputted, where *µ* denotes the mean, and *σ* denotes the standard deviation; and when the number of the comparison results indicating that the deviation range (*µ* - 2*σ*, *µ* + 2*σ*) is exceeded is greater than or equal to the preset quantity threshold, the out-of-control indicator is outputted.

Optionally, the preset correspondence refers to the correspondence between the number of the comparison results indicating that the deviation range is exceeded and a candidate abnormality indicator.

For specific processes of S401-S406, reference may be made to the descriptions in the above method embodiments, and the implementation principles and technical effects thereof are similar and will not be repeatedly described herein.

It should be understood that although the steps in the flowcharts of the above embodiments are shown sequentially as indicated by the arrows, the steps are not necessarily executed in this exact order. Unless explicitly stated herein, there is no strict sequential limitation on the execution of these steps, and these steps may be performed in other orders. Furthermore, at least some of the steps in the flowcharts of the above embodiments may include multiple steps or stages. These steps or stages are not necessarily completed at the same time but can be executed at different times, and the execution order of these steps or stages is not necessarily sequential but can be performed alternately or in turn with other steps or parts of sub-steps or stages of other steps.

Based on the same inventive concept, embodiments of this application also provide an apparatus for determining instrument status, which is used to implement the method for determining instrument status above. The problem-solving implementations provided by the apparatus are similar to those described in the method above, therefore, for specific limitations in one or more embodiments of the apparatus for determining instrument status provided below, reference can be made to the limitations of the method for determining instrument status above, which will not be repeatedly described herein.

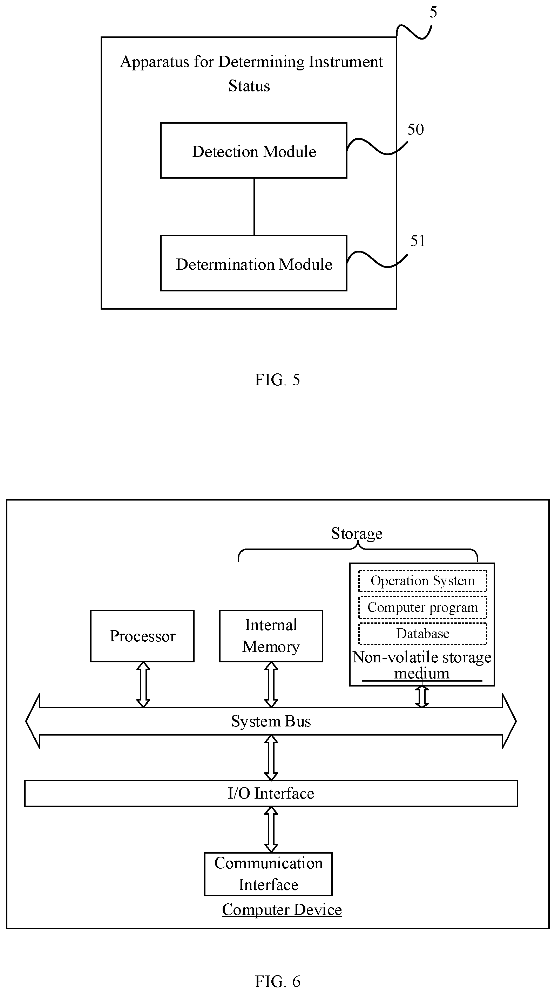

In an embodiment, FIG. 5 is a block diagram showing an apparatus for determining instrument status according to an embodiment. As shown in FIG. 5, the apparatus 5 includes a detection module 50 and a determination module 51.

The detection module 50 is configured to, in a case where a self-QC reagent is installed in a target instrument, perform a self-QC test on the target instrument using the self-QC reagent to obtain a target test result of the self-QC test.

The determination module 51 is configured to determine a status of the target instrument based on the target test result of the self-QC test.

In the above apparatus for determining instrument status, in the case where the self-QC reagent is installed in the target instrument, the self-QC test is performed on the target instrument using the self-QC reagent to obtain the target test result of the self-QC test, and then the status of the target instrument is determined based on the target test result of the self-QC test. Compared with the in vitro diagnostic analyzers in the prior art, which rely on the cumbersome manual QC procedures to determine the instrument status thus resulting the lower accuracy and reliability of the determined instrument status, the apparatus for determining instrument status of the present application, in which the self-QC reagent installed in the instrument is used to automatically perform the self-QC test on the target instrument and the entire process of the detection is automatically executed by a computer, greatly improves the accuracy and reliability of the ultimately determined instrument status.

In an embodiment, the determination module 51 is specifically configured to:
in the case where the target test result of the self-QC test follows the Westgard rule, determine the status of the target instrument to be normal; and in the case where the target test result of the self-QC test does not follow the Westgard rule, determine the status of the target instrument to be abnormal.

In an embodiment, the apparatus 5 further includes:
an acquisition module configured to obtain the mean of multiple historical test results of the self-QC test in the case where the target test result of the self-QC test does not follow the Westgard rule;
a comparison module configured to compare the target test results of the self-QC test with the mean to obtain multiple comparison results; and
an output module configured to output a target abnormality indicator based on the multiple comparison results.

In an embodiment, the output module specifically includes:
an acquisition unit configured to obtain multiple deviation ranges, where the multiple deviation ranges are determined based on a standard deviation of the mean;
an output unit configured to output the target abnormality indicator based on the number of the comparison results indicating that the deviation range is exceeded and a preset correspondence, where the correspondence refers to the correspondence between the number of the comparison results indicating that the deviation range is exceeded and a candidate abnormality indicator.

In an embodiment, the output unit is specifically configured to:
output the warning indicator when the number of the comparison results indicating that the deviation range (*µ* - 2*σ*, *µ* + 2*σ*) is exceeded is less than a preset quantity threshold, where *µ* denotes the mean, and *σ* denotes the standard deviation; and output the out-of-control indicator when the number of the comparison results indicating that the deviation range (*µ* - 2*σ*, *µ* + 2*σ*) is exceeded is greater than or equal to the preset quantity threshold.

In an embodiment, the determination module 51 is specifically configured to:
perform the self- QC test on the target instrument by adopting the preset method and using the self-QC reagent to obtain the target test result of the self-QC test, where the preset method is one of a competitive method, a sandwich method, or an indirect method, and preferably, the preset method is the competitive method.

The various modules in the above apparatus for determining instrument status may be implemented wholly or partially by software, hardware, or a combination thereof. The above modules may be embedded in or independent of the processor within the computer device in the form of hardware, or they may be stored in the memory of the computer device in the form of software, thereby making it convenient for the processor to call and execute the operations corresponding to the above modules.

In an embodiment, a computer device is provided. The computer device may be a server, and an internal structural diagram thereof can be as shown in FIG. 6. The computer device includes a processor, a memory, a network interface, and a transceiver connected via a system bus. The processor of the computer device is configured to provide computing and control capabilities. The memory of the computer device includes a non-volatile storage medium and an internal memory. The transceiver of the computer device is configured to execute the operations of receiving or sending data under the control of the processor. The non-volatile storage medium stores an operating system, computer programs, and a database. The internal memory provides an environment for the operation of the operating system and computer programs in the non-volatile storage medium. The database of the computer device is configured to store data such as sample data. The network interface of the computer device is configured to communicate with external terminals via a network connection. The computer program, when executed by the processor, implements a method for determining instrument status.

Those skilled in the art can understand that the structural diagram shown in FIG. 6 is merely a block diagram of a partial structure related to the solution of this application and does not constitute a limitation on the computer device to which the solution of this application is applied. Specifically, the computer device may include more or fewer components than those shown in the figure, or some combined components, or a different component arrangement.

In an embodiment, a computer device is provided, including a memory and a processor. The memory has a computer program stored thereon, and the processor, when executing the computer program, implements the following steps of:
in the case where the self-QC reagent is installed in the target instrument, performing the self-QC test on the target instrument using the self-QC reagent to obtain the target test result of the self-QC test; and
determining a status of the target instrument based on the target test result of the self-QC test.

In an embodiment, the processor, when executing a logic in the computer program of determining the status of the target instrument based on the target test result of the self-QC test, implements the following steps of:
in the case where the target test result of the self-QC test follows the Westgard rule, determining the status of the target instrument to be normal; and in the case where the target test result of the self-QC test does not follow the Westgard rule, determining the status of the target instrument to be abnormal.

In an embodiment, the processor, when executing the logic in the computer program, further implements the following steps of:
in the case where the target test result does not follow the Westgard rule, obtaining the mean of multiple historical test results of the self-QC test; comparing the target test results of the self-QC test with the mean to obtain multiple comparison results; and outputting a target abnormality indicator based on the multiple comparison results.

In an embodiment, the processor, when executing the logic in the computer program of outputting the target abnormality indicator based on the multiple comparison results, further implements the following steps of:
obtaining multiple deviation ranges, where the multiple deviation ranges are determined based on the standard deviation of the mean; and outputting the target abnormality indicator based on the preset correspondence and the number of the comparison results indicating that the deviation range is exceeded, where the preset correspondence refers to the correspondence between the number of the comparison results indicating that the deviation range is exceeded, and a candidate abnormality indicator.

In an embodiment, the candidate abnormality indicator includes a warning indicator and an out-of-control indicator. The processor, when executing the logic in the computer program of outputting the target abnormality indicator based on the preset correspondence and the number of the comparison results indicating that the deviation range is exceeded, further implements the following steps of:
outputting the warning indicator when the number of the comparison results indicating that the deviation range (*µ* - 2*σ*, *µ* + 2*σ*) is exceeded is less than a preset quantity threshold, where *µ* denotes the mean, and *σ* denotes the standard deviation; and outputting the out-of-control indicator when the number of the comparison results indicating that the deviation range (*µ* - 2*σ*, *µ* + 2*σ*) is exceeded is greater than or equal to the preset quantity threshold.

In an embodiment, the processor, when executing the logic in the computer program of performing the self- QC test on the target instrument using the self-QC reagent to obtain the target test result of the self-QC test, further implements the following steps of:
performing the self-QC test on the target instrument by adopting the preset method and using the self-QC reagent to obtain the target test result of the self-QC test, where the preset method is one of a competitive method, a sandwich method, or an indirect method, and preferably, the preset method is the competitive method.

For the principles and specific processes of the above-provided computer device in implementing the various embodiments, reference may be made to the descriptions in the aforementioned embodiments of the method for determining instrument status, which will not be repeatedly described herein.

In an embodiment, a computer-readable storage medium is provided, having a computer program stored thereon. The computer program, when executed by a processor, causes the processor to implement the following steps of:
in the case where the self-QC reagent is installed in the target instrument, performing the self-QC test on the target instrument using the self-QC reagent to obtain the target test result of the self-QC test; and
determining a status of the target instrument based on the target test result of the self-QC test.

In an embodiment, the logic in the computer program of determining the status of the target instrument based on the target test result of the self-QC test, when executed by the processor, causes the processor to specifically implement steps of:
in the case where the target test result of the self-QC test follows the Westgard rule, determining the status of the target instrument to be normal; and in the case where the target test result of the self-QC test does not follow the Westgard rule, determining the status of the target instrument to be abnormal.

In an embodiment, the logic in the computer program, when executed by the processor, causes the processor to specifically implement steps of:
in the case where the target test result of the self-QC test does not follow the Westgard rule, obtaining the mean of multiple historical test results of the self-QC test; comparing the target test results of the self-QC test with the mean to obtain multiple comparison results; and outputting a target abnormality indicator based on the multiple comparison results.

In an embodiment, the logic in the computer program of outputting the target abnormality indicator based on the multiple comparison results, when executed by the processor, causes the processor to specifically implement steps of:
obtaining multiple deviation ranges, where the multiple deviation ranges are determined based on the standard deviation of the mean; and outputting the target abnormality indicator based on the preset correspondence and the number of the comparison results indicating that the deviation range is exceeded, where the preset correspondence refers to the correspondence between the number of the comparison results indicating that the deviation range is exceeded and a candidate abnormality indicator.

In an embodiment, the candidate abnormality indicator includes a warning indicator and an out-of-control indicator. The logic in the computer program of outputting the target abnormality indicator based on the preset correspondence and the number of the comparison results indicating that the deviation range is exceeded, when executed by the processor, causes the processor to specifically implement steps of:
outputting the warning indicator when the number of the comparison results indicating that the deviation range (*µ* - 2*σ*, *µ* + 2*σ*) is exceeded is less than a preset quantity threshold, where *µ* denotes the mean, and *σ* denotes the standard deviation; and outputting the out-of-control indicator when the number of the comparison results indicating that the deviation range (*µ* - 2*σ*, *µ* + 2*σ*) is exceeded is greater than or equal to the preset quantity threshold.

In an embodiment, the logic in the computer program of performing the self- QC test on the target instrument using the self-QC reagent to obtain the target test result of the self-QC test, when executed by the processor, causes the processor to specifically implement steps:
performing the self-QC test on the target instrument by adopting the preset method and using the self-QC reagent to obtain the target test result of the self-QC test, where the preset method is one of a competitive method, a sandwich method, or an indirect method, and preferably, the preset method is the competitive method.

For the principles and specific processes of the above-provided computer-readable storage medium in implementing the various embodiments, reference may be made to the descriptions in the aforementioned embodiments of the target detection method, which will not be repeatedly described herein.

In an embodiment, a computer program product is provided, including a computer program. The computer program, when executed by a processor, implements the following steps of:
in the case where the self-QC reagent is installed in the target instrument, performing the self-QC test on the target instrument using the self-QC reagent to obtain the target test result of the self-QC test; and
determining a status of the target instrument based on the target test result of the self-QC test.

In an embodiment, the logic in the computer program of determining the status of the target instrument based on the target test result of the self-QC test, when executed by the processor, specifically implements steps of:
in the case where the target test result of the self-QC test follows the Westgard rule, determining the status of the target instrument to be normal; and in the case where the target test result of the self-QC test does not follow the Westgard rule, determining the status of the target instrument to be abnormal.

In an embodiment, the logic in the computer program, when executed by the processor, specifically implements steps of:
in the case where the target test result of the self-QC test does not follow the Westgard rule, obtaining the mean of multiple historical test results of the self-QC test; comparing the target test results of the self-QC test with the mean to obtain multiple comparison results; and outputting a target abnormality indicator based on the multiple comparison results.

In an embodiment, the logic in the computer program of outputting the target abnormality indicator based on the multiple comparison results, when executed by the processor, specifically implements steps of:
obtaining multiple deviation ranges, where the multiple deviation ranges are determined based on the standard deviation of the mean; and outputting the target abnormality indicator based on the preset correspondence and the number of the comparison results indicating that the deviation range is exceeded, where the preset correspondence refers to the correspondence between the number of the comparison results indicating that the deviation range is exceeded and a candidate abnormality indicator.

In an embodiment, the candidate abnormality indicator includes a warning indicator and an out-of-control indicator. The logic in the computer program of outputting the target abnormality indicator based on the preset correspondence and the number of the comparison results indicating that the deviation range is exceeded, when executed by the processor, specifically implement steps of:
outputting the warning indicator when the number of the comparison results indicating that the deviation range (*µ* - 2*σ*, *µ* + 2*σ*) is exceeded is less than a preset quantity threshold, where *µ* denotes the mean, and *σ* denotes the standard deviation; and outputting the out-of-control indicator when the number of the comparison results indicating that the deviation range (*µ* - 2*σ*, *µ* + 2*σ*) is exceeded is greater than or equal to the preset quantity threshold.

In an embodiment, the logic in the computer program of performing the self- QC test on the target instrument using the self-QC reagent to obtain the target test result of the self-QC test, when executed by the processor, specifically implements steps of:
performing the self-QC test on the target instrument by adopting the preset method and using the self-QC reagent to obtain the target test result of the self-QC test, where the preset method is one of a competitive method, a sandwich method, or an indirect method, and preferably, the preset method is the competitive method.

For the principles and specific processes of the above-provided computer program product in implementing the various embodiments, reference may be made to the descriptions in the aforementioned embodiments of the target detection method, which will not be repeated here.

It should be noted that the information involved in the application (including but not limited to the target test results, the status information of the target instrument, etc., in the method for determining instrument status in the application) has been fully authorized by all parties.

Those of ordinary skill in the art can understand that all or part of the processes in the methods of the above embodiments can be implemented by a computer program instructing relevant hardware. The computer program may be stored in a non-volatile computer-readable storage medium. The computer program, when executed, may include the processes of the embodiments of the above methods. Any reference to memory, database, or other media used in the embodiments provided in this application may include at least one of non-volatile or volatile memory. Non-volatile memory may include Read-Only Memory (ROM), magnetic tape, floppy disk, flash memory, optical memory, high-density embedded non-volatile memory, Resistive Random Access Memory (ReRAM), Magnetoresistive Random Access Memory (MRAM), Ferroelectric Random Access Memory (FRAM), Phase Change Memory (PCM), graphene memory, etc. Volatile memory may include Random Access Memory (RAM) or external cache memory, etc. By way of illustration and not limitation, RAM can be in various forms, such as Static Random Access Memory (SRAM) or Dynamic Random Access Memory (DRAM). The databases involved in the embodiments provided in this application may include at least one of relational databases and non-relational databases. Non-relational databases may include blockchain-based distributed databases, etc., but are not limited thereto. The processors involved in the embodiments provided in this application may be general-purpose processors, central processing units, graphics processing units, digital signal processors, programmable logic devices, quantum computing-based data processing logic devices, etc., but are not limited thereto.

The various technical features of the above embodiments can be combined arbitrarily. To make the description concise, not all possible combinations of the various technical features in the above embodiments are described. However, as long as there is no contradiction in the combinations of these technical features, these combinations should be considered within the scope recorded in this specification.

The above embodiments merely express several implementation modes of this application, and the descriptions thereof are relatively specific and detailed but should not thereby be construed as limiting the scope of the patent of this application. It should be noted that for those of ordinary skill in the art, several modifications and improvements can also be made without departing from the concept of this application, and these modifications and improvements fall within the protection scope of this application. Therefore, the protection scope of the patent of this application shall be subject to the appended claims.

## Claims

1. A method for determining instrument status, **characterized by** comprising:
performing a self-quality control (QC) test on a target instrument using a self-quality control (QC) reagent to obtain a target test result of the self-QC test, in a case where the self-QC reagent is installed in the target instrument; and
determining a status of the target instrument based on the target test result of the self-QC test.

2. The method according to claim 1, wherein determining the status of the target instrument based on the target test result of the self-QC test comprises:
determining the status of the target instrument to be normal, in a case where the target test result of the self-QC test follows a Westgard rule; and
determining the status of the target instrument to be abnormal, in a case where the target test result of the self-QC test does not follow the Westgard rule.

3. The method according to claim 2, wherein further comprising:
obtaining a mean of multiple historical test results of the self-QC test, in the case where the target test result of the self-QC test does not follow the Westgard rule;
comparing the target test result of the self-QC test with the mean to obtain multiple comparison results; and
outputting a target abnormality indicator based on the multiple comparison results.

4. The method according to claim 3, wherein outputting the target abnormality indicator based on the multiple comparison results comprises:
obtaining multiple deviation ranges, the multiple deviation ranges being determined based on a standard deviation of the mean; and
outputting the target abnormality indicator based on a preset correspondence and the number of comparison results indicating that the deviation range is exceeded, wherein the preset correspondence refers to a correspondence between the number of the comparison results indicating that the deviation range is exceeded, and a candidate abnormality indicator.

5. The method according to claim 4, wherein the candidate abnormality indicator comprises a warning indicator and an out-of-control indicator; and outputting the target abnormality indicator based on the preset correspondence and the number of the comparison results indicating that the deviation range is exceeded comprises:
outputting the warning indicator when the number of the comparison results indicating that the deviation range (*µ* - 2*σ*, *µ* + 2*σ*) is exceeded is less than a preset quantity threshold, wherein *µ* denotes the mean, and *σ* denotes the standard deviation; and
outputting the out-of-control indicator when the number of the comparison results indicating that the deviation range (*µ* - 2*σ*, *µ* + 2*σ*) is exceeded is greater than or equal to the preset quantity threshold.

6. The method according to claim 1, wherein performing the self-QC test on the target instrument using the self-QC reagent to obtain the target test result of the self-QC test comprises:
performing the self- QC test on the target instrument by adopting a preset method and using the self-QC reagent to obtain the target test result of the self-QC test, wherein the preset method is one of a competitive method, a sandwich method, or an indirect method.

7. An apparatus for determining instrument status, **characterized by** comprising:
a detection module configured to perform a self-quality control (QC) test on a target instrument using a self-quality control (QC) reagent to obtain a target test result of the self-QC test, in a case where the self-QC reagent is installed in the target instrument; and
a determination module configured to determine a status of the target instrument based on the target test result of the self-QC test.

8. A computer device, comprising a memory and a processor, wherein the memory has a computer program stored thereon, and the processor, when executing the computer program, implements steps of the method according to any one of claims 1 to 6.

9. A computer-readable storage medium, having a computer program stored thereon, wherein the computer program, when executed by a processor, causes the processor to implement steps of the method according to any one of claims 1 to 6.

10. A computer program product, comprising a computer program, wherein the computer program, when executed by a processor, implements steps of the method according to any one of claims 1 to 6.
